(19) Europäisches Patentamt
European Patent Office
Office européen des brevets

(11) **EP 2 804 120 A2**

(12) **EUROPEAN PATENT APPLICATION**

(43) Date of publication:
**19.11.2014 Bulletin 2014/47**

(51) Int Cl.:
**G06F 19/00** (2011.01)

(21) Application number: **14168540.4**

(22) Date of filing: **15.05.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **17.05.2013 JP 2013104665**

(71) Applicant: **Hitachi Ltd.**
**Tokyo (JP)**

(72) Inventors:
• **Hasegawa, Yasutaka**
  **Chiyoda-ku, Tokyo 100-8280 (JP)**
• **Ban, Hideyuki**
  **Chiyoda-ku, Tokyo 100-8280 (JP)**
• **Tomita, Naofumi**
  **Chiyoda-ku, Tokyo 100-8280 (JP)**

(74) Representative: **Moore, Graeme Patrick et al**
**Mewburn Ellis LLP**
**33 Gutter Lane**
**London**
**EC2V 8AS (GB)**

(54) **Analysis System and Analysis Method**

(57) It is provided an analysis system comprising: an input unit to receive a medical cost of a insured person, intervention information on a provision of an intervention service and a start date of the intervention service; a propensity score calculation unit to analyze a relationship between the medical cost before the provision of the intervention service and the intervention information, and to calculate a propensity score of an intervention group and a propensity score of a nonintervention group; and an adjusted medical cost calculation unit to calculate adjusted medical costs of the intervention group before and after the provision of the intervention service by using the propensity score of the intervention group and medical costs of the intervention group before and after the provision of the intervention service, and to calculate adjusted medical costs of the nonintervention group before and after the provision of the intervention service.

Fig. 1

EP 2 804 120 A2

**Description**

CLAIM OF PRIORITY

The present application claims priority from Japanese patent application JP 2013-104665 filed on May 17, 2013, the content of which is hereby incorporated by reference into this application.

BACKGROUND OF THE INVENTION

This invention relates to an analysis system for analyzing an effect of a healthcare guidance business.

An insurer (health insurance society) provides a healthcare service such as a healthcare guidance in order to adjust a medical cost, and it is thus important to analyze an effect of the healthcare service on the medical cost after the provision. For example, in Japanese Patent Application Laid-open No. 2004-341611 A, there is disclosed an insurer information system including: a unified database server including an insured person database for storing health management information on the insured persons; an intervention determination support apparatus for reading the health management information on the insured persons from the insured person database, determining intervention subject information on intervention subjects requiring an intervention in health management from the insured persons, and determining intervention support information for supporting health promotion of the intervention subjects; and a result evaluation apparatus for inputting application result data representing a result of application to the intervention subject based on the intervention support information and the health management information to evaluate the intervention support information determined by the intervention determination support apparatus.

Moreover, as a method of analyzing the effect of the intervention, there is proposed a method of adjusting background information (covariance) of an intervention group and a nonintervention group by using a propensity score for comparison and analysis.

SUMMARY OF THE INVENTION

With the method disclosed in Japanese Patent Application Laid-open No. 2004-341611 A, distinguishment between the intervention effect and an influence due to a temporal change from each other is not available, resulting in difficulty in the analysis of the effect of the intervention on the medical cost. In other words, comparison with the nonintervention group is necessary for the analysis of the intervention effect, but in Japanese Patent Application Laid-open No. 2004-341611 A, there is not disclosed that the analysis of the effect on the medical cost is carried out by using the comparison with the nonintervention group.

One embodiment of this invention provides a healthcare service effect analysis system for analyzing an effect of a healthcare guidance business on a medical cost based on healthcare cost information and healthcare guidance business information held by a health insurance society.

The representative one of inventions disclosed in this application is outlined as follows. There is provided an analysis system for executing a program to analyze an effect of a healthcare guidance business, comprising: a processor for executing the program; a memory for storing the program ; an input unit configured to control the processor to receive a medical cost of a insured person, intervention information on a provision of an intervention service and a start date of the intervention service; a propensity score calculation unit configured to control the processor to analyze a relationship between the medical cost before the provision of the intervention service and the intervention information, and to calculate a propensity score of an intervention group representing that the intervention service is provided and a propensity score of a nonintervention group representing that the intervention service is not provided from the medical cost before the provision of the intervention service; and an adjusted medical cost calculation unit configured to control the processor to calculate adjusted medical costs of the intervention group before and after the provision of the intervention service by using the propensity score of the intervention group and medical costs of the intervention group before and after the provision of the intervention service, and to calculate adjusted medical costs of the nonintervention group before and after the provision of the intervention service by using the propensity score of the nonintervention group and medical costs of the nonintervention group before and after the provision of the intervention service.

According to representative embodiment of this invention, the medical costs for each of diseases of the intervention group and the nonintervention group can be accurately compared with each other. Problems, configurations, and effects which have not been described become apparent from the following description of embodiments.

BRIEF DESCRIPTION OF THE DRAWINGS

The present invention can be appreciated by the description which follows in conjunction with the following figures, wherein:

FIG. 1 is a configuration diagram of a healthcare service effect analysis system according to this embodiment of this invention;

FIG. 2 is an explanatory diagram illustrating an example of insured person information managed by an insured person information management unit;

FIG. 3 is an explanatory diagram illustrating an example of intervention information managed by an intervention information management unit;

FIG. 4 is an explanatory diagram illustrating an example of healthcare cost information managed by a healthcare cost information management unit;

FIG. 5 is an explanatory diagram illustrating an example of data for analysis information managed by a data-for-analysis management unit;

FIG. 6 is a flowchart of processing of generating data for analysis;

FIG. 7 is an explanatory diagram illustrating an example of propensity score calculation equations managed by a propensity score calculation equation management unit;

FIG. 8 is an explanatory diagram illustrating an example of propensity scores per disease managed by a propensity score per disease management unit;

FIG. 9 is a flowchart of processing of calculating medical cost effect from the data for analysis;

FIG. 10 is an explanatory diagram illustrating an example of a healthcare information input screen;

FIG. 11 is an explanatory diagram illustrating an example of a healthcare service effect display screen;

FIG. 12 is a flowchart of adjusted medical cost calculation processing considering a average medical cost difference between each of intervention groups and a nonintervention group;

FIG. 13 is an explanatory diagram illustrating an example of health checkup information managed by a health checkup information management unit; and

FIG. 14 is a flowchart of an adjusted medical cost calculation processing considering health checkup information.

## DETAILED DESCRIPTION OF THE PREFERRED EMBODIMENTS

Now, a description is given of embodiments of this invention referring to the drawings.

[First embodiment]

A healthcare service effect analysis system according to an embodiment of this invention is a computer system including a processor (CPU), a memory, and a storage medium. Moreover, the healthcare service effect analysis system according to this embodiment may be a computer system constructed by a single computer, or a computer system constructed by a server and client terminals.

The storage medium is, for example, a nonvolatile storage medium. The nonvolatile storage medium is, for example, a magnetic disk or a nonvolatile memory. The nonvolatile storage medium stores programs for realizing functions of the healthcare service effect analysis system, and calculation results thereof, and the like. The programs stored in the storage medium are deployed on the memory. The CPU executes the programs deployed on the memory. Processing and arithmetic operations described later are carried out by the CPU.

The healthcare service effect analysis system is a computer system constructed on a single computer, or on a plurality of logically or physically constructed computers, and may operate as independent threads on the same computer, or may operate on virtual computers constructed on a plurality of physical computer resources.

The program executed by the processor is provided for respective servers by means of a removable medium (such as a CD-ROM and a flash memory), and is stored in a non-volatile storage apparatus which is a non-temporary recording medium. Therefore, the computer system preferably includes an interface for reading the removable medium.

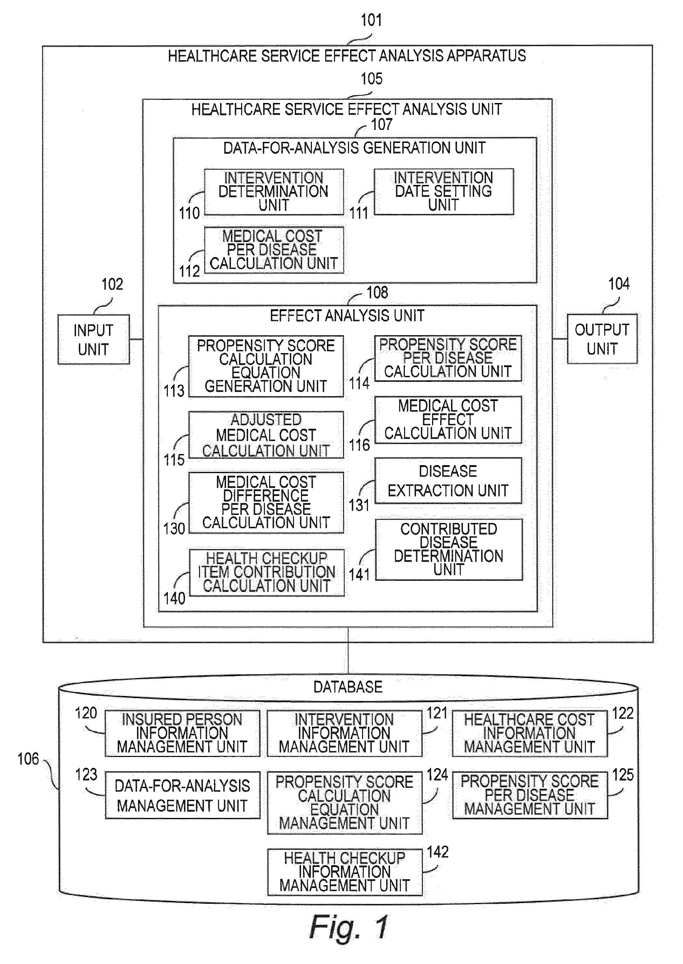

FIG. 1 is a configuration diagram of the healthcare service effect analysis system according to this embodiment.

The healthcare service effect analysis system includes a healthcare service effect analysis apparatus 101 and a database 106.

The healthcare service effect analysis apparatus 101 includes an input unit 102, a healthcare service effect analysis unit 105, and an output unit 104. The input unit 102 is a human interface such as a mouse and a keyboard, and receives an input to the healthcare service effect analysis apparatus 101. The output unit 104 includes a display and a printer for outputting an arithmetic operation result obtained by the healthcare service effect analysis apparatus 101.

The healthcare service effect analysis unit 105 is implemented by the processor executing a predetermined program, and includes a data-for-analysis generation unit 107 and an effect analysis unit 108.

The data-for-analysis generation unit 107 includes an intervention determination unit 110, an intervention date setting unit 111, and a medical cost per disease calculation unit 112.

The intervention determination unit 110 identifies a insured person to which the intervention has been carried out based on insured person information of a health insurance and intervention information input to the input unit 102, and assigns a flag representing an intervention group or a nonintervention group to the insured person. If intervention information on a plurality of intervention services exists, the flag representing the intervention group is assigned to the respective services.

The intervention date setting unit 111 acquires intervention start dates from the intervention information for the intervention group, and sets intervention dates. Moreover, the intervention setting unit 111 calculates a distribution of the intervention start dates of the intervention group, and randomly sets the intervention dates for the nonintervention group so that distributions of the intervention start dates are equal to each other between the intervention group and the nonintervention group.

The medical cost per disease calculation unit 112 calculates an annual medical cost per disease before the intervention and an annual medical cost per disease after the intervention based on the insured person information of the health insurance and healthcare cost information input to the input unit 102 and the intervention date set by the intervention date setting unit 111.

The data-for-analysis generation unit 107 generates, for each insured person, data for analysis including the flag representing each of the intervention groups and the flag representing the nonintervention group for each intervention service assigned by the intervention determination unit 110, the intervention dates set by the intervention date setting unit 111, and the medical costs per disease before and after the intervention calculated by the medical cost per disease calculation unit 112.

The effect analysis unit 108 includes a propensity score calculation equation generation unit 113, a propensity score per disease calculation unit 114, an adjusted medical cost calculation unit 115, and a medical cost effect calculation unit 116. Moreover, the effect analysis unit 108 may include a medical cost difference per disease calculation unit 130, a disease extraction unit 131, a health checkup item contribution calculation unit 140, and a contributed disease determination unit 141. The medical cost difference per disease calculation unit 130, the disease extraction unit 131, the health checkup item contribution calculation unit 140, and the contributed disease determination unit 141 are configurations used for a modified example described later, and a description thereof is given later.

The propensity score calculation equation generation unit 113 acquires the data for analysis generated by the data-for-analysis generation unit 107, analyzes a relationship between the medical cost per disease before the intervention and the intervention service and a relationship between the medical cost per disease before the intervention and the nonintervention, and generates an equation for calculating a propensity score P for each intervention service and each nonintervention group. A description is later given of a specific generation method.

The propensity score per disease calculation unit 114 decomposes the propensity score calculation equation generated by the propensity score calculation equation generation unit 113 into equations for each of the diseases, and calculates propensity scores per disease. A description is later given of a specific calculation method.

The adjusted medical cost calculation unit 115 calculates adjusted medical costs per disease before and after the intervention adjusted by the propensity score per disease calculated by the propensity score per disease calculation unit 114 for each of the intervention services and the nonintervention group.

The medical cost effect calculation unit 116 subtracts the adjusted medical cost per disease after the intervention of the intervention service from the adjusted medical cost per disease after the intervention of the nonintervention group calculated by the adjusted medical cost calculation unit 115, thereby calculating a medical cost restraint effect for each of the intervention services.

The effect analysis unit 108 displays, on the output unit 104, the adjusted medical cost per disease for each of the intervention services and the nonintervention group calculated by the adjusted medical cost calculation unit 115 and the medical cost restraint effect for each of the intervention services calculated by the medical cost effect calculation unit 116.

The database 106 stores an insured person information management unit 120, an intervention information management unit 121, a healthcare cost information management unit 122, a data-for-analysis management unit 123, a propensity score calculation equation management unit 124, and a propensity score per disease management unit 125. Moreover, the database 106 may store a health checkup information management unit 142. The health checkup information management unit 142 is a configuration used for the modified example described later, and a description thereof is therefore given later.

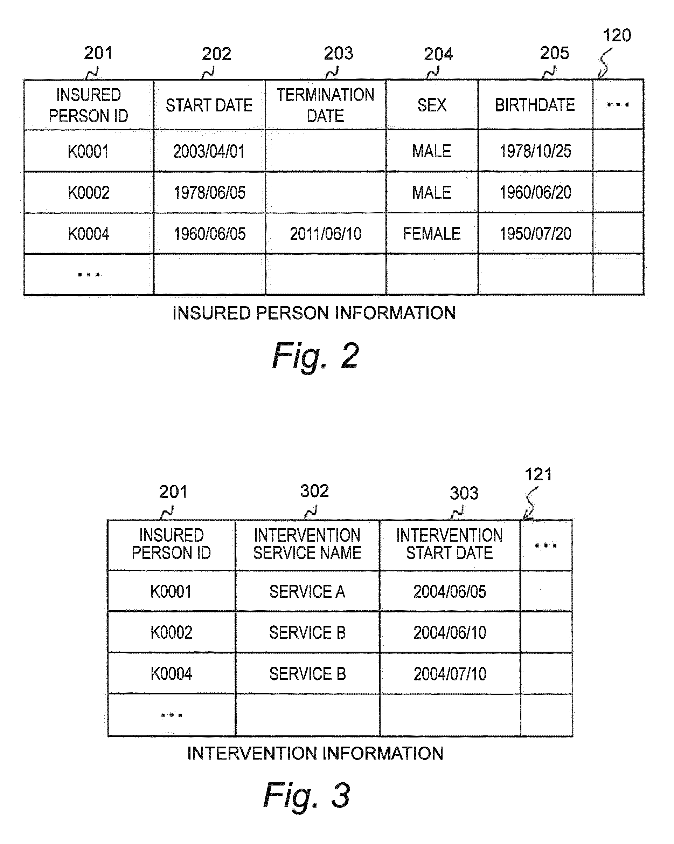

The insured person management unit 120 manages information on the insured persons input to the input unit 102. Referring to FIG. 2, a description is given of a configuration example of the insured person information management unit 120.

The intervention information management unit 121 manages intervention information for each of the insured persons input to the input unit 102. Referring to FIG. 3, a description is given of a configuration example of the intervention information management unit 121.

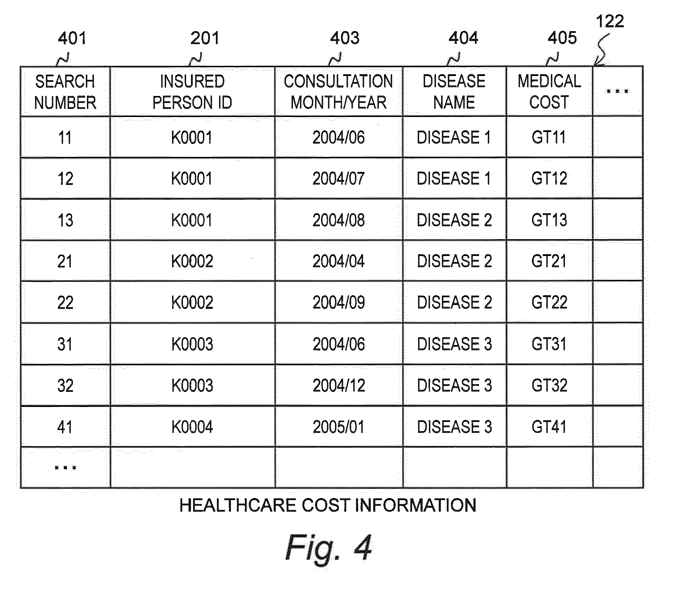

The healthcare cost information management unit 122 manages medical presentation information on the insured persons input to the input unit 102. Referring to FIG. 4, a description is given of a configuration example of the healthcare cost information management unit 122.

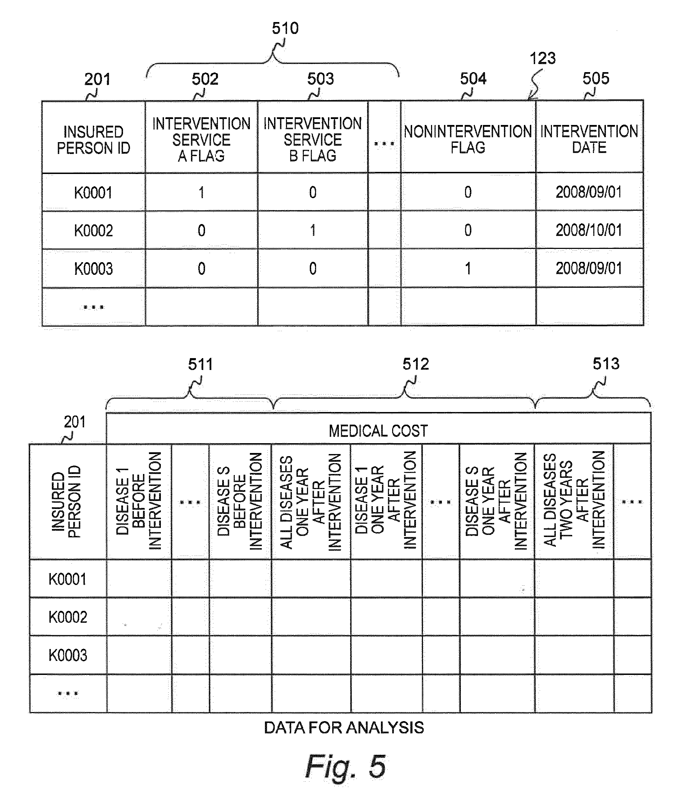

The data-for-analysis management unit 123 manages the data for analysis generated by the data-for-analysis generation unit 107 based on the insured person information of FIG. 2, the intervention information of FIG. 3, and the healthcare cost information of FIG. 4. The data for analysis is data used for analyzing the effect on the medical cost. Referring to FIG. 5, a description is given of a configuration example of the data-for-analysis management unit 123.

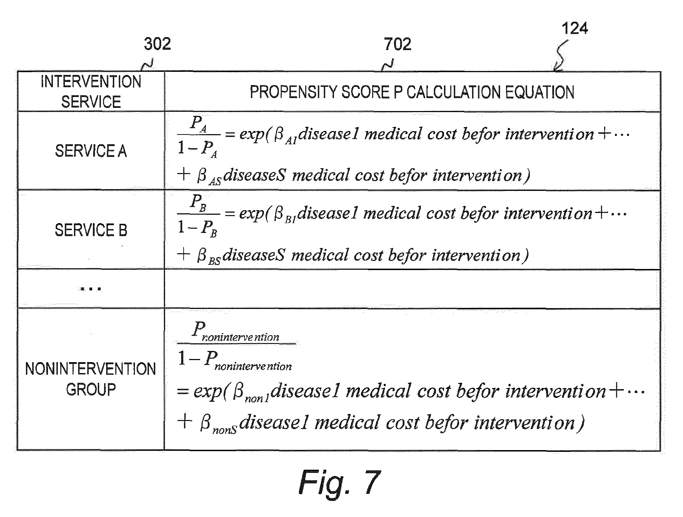

The propensity score calculation equation management unit 124 manages the propensity score calculation equation for each of the intervention services generated by the propensity score calculation equation generation unit 113. Referring to FIG. 7, a description is given of a configuration example of the propensity score calculation equation management unit 124.

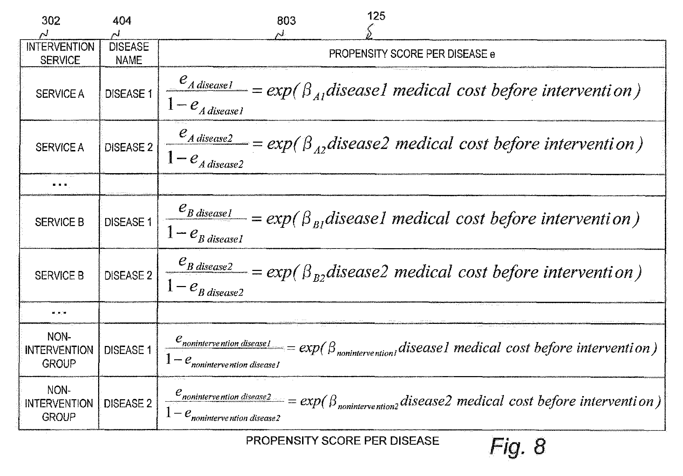

The propensity score per disease management unit 125 manages the propensity score per disease for each of the intervention services and for each of the diseases calculated by the propensity score per disease calculation unit 114. Referring to FIG. 8, a description is given of a configuration example of the propensity score per disease management unit 125.

FIG. 2 is an explanatory diagram illustrating an example of the insured person information managed by the insured person information management unit 120.

The insured person information management unit 120 includes data such as insured person IDs 201, start dates 202, termination dates 203, sex 204, and birthdates 205. The insured person ID 201 is identification information for identifying an insured person. The start date 202 and the termination date 203 are respectively a date when the insured person takes out the health insurance and a date when the insured person terminates the health insurance. The sex 204 and the birthdate 205 are respectively the sex and birthdate of the insured person.

FIG. 3 is an explanatory diagram illustrating an example of the intervention information managed by the intervention information management unit 121.

The intervention information management unit 121 includes data such as insured person IDs 201, intervention service names 302, and intervention start dates 303. The intervention service name 302 is a name of an intervention service provided for the insured person. The intervention start date 303 is a date, month, and year when the intervention service was started.

FIG. 4 is an explanatory diagram illustrating an example of the healthcare cost information managed by the healthcare cost information management unit 122.

The healthcare cost information management unit 122 includes data such as search numbers 401, insured person IDs 201, consultation onths/years 403, disease names 404, and medical costs 405. The search number 401 is an identifier for identifying one healthcare cost. The consultation month/year 403 is a month and year when the insured person has received a clinical action. The disease name 404 is the name of a disease for which the insured person has received the clinical action. The medical cost 405 is a cost spent for the clinical action.

FIG. 5 is an explanatory diagram illustrating an example of the data for analysis information managed by the data-for-analysis management unit 123.

The data-for-analysis management unit 123 includes data such as insured person IDs 201, intervention service flags 510, nonintervention flags 504, intervention dates 505, medical costs per disease before intervention 511, medical costs per disease one year after intervention 512, and medical costs per disease two years after intervention 513. The intervention service flag 510 is a flag representing whether each intervention service has been provided or not (502 and 503). The nonintervention flag 504 is a flag representing a state where no intervention service is provided (nonintervention state). The intervention date 505 is a date, month, and year when the intervention service started. Each of the medical cost per disease before intervention 511, the medical cost per disease one year after intervention 512, and the medical cost per disease two years after intervention 513 is a medical cost for each disease in a corresponding period.

FIG. 7 is an explanatory diagram illustrating an example of the propensity score calculation equations managed by the propensity score calculation equation management unit 124.

The propensity score calculation equation management unit 124 includes intervention services 302 and propensity score calculation equations 702. The propensity score calculation equation 702 is a propensity score calculation equation for an intervention service (for each of the intervention services and the nonintervention group). The propensity score calculation equation is, as described later, an equation for analyzing a relationship between the medical cost for each of the diseases before the intervention and each of the intervention services, or a relationship between the medical cost for each of the diseases before the intervention and the nonintervention, thereby calculating the propensity score P.

FIG. 8 is an explanatory diagram illustrating an example of the propensity scores per disease managed by the propensity score per disease management unit 125.

The propensity score per disease management unit 125 includes intervention services 302, disease names 404, and propensity scores per disease 803. The propensity score per disease 803 is a propensity score per disease for each of the intervention services (for each of the intervention services and the nonintervention group) and for each of the disease names 404.

A description is now given of processing of generating the data for analysis based on the input insured person information, intervention information, and healthcare cost information by the healthcare service effect analysis apparatus 101 according to this embodiment.

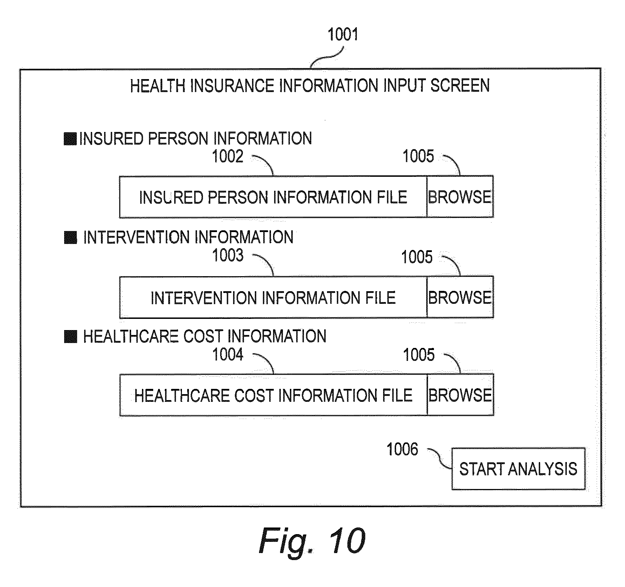

FIG. 10 is an explanatory diagram illustrating an example of a healthcare information input screen 1001 displayed by the healthcare service effect analysis apparatus 101 on the unit 104.

The healthcare information input screen 1001 receives an input of files from which the insured person information, the intervention information, and the healthcare cost information are acquired.

Specifically, the healthcare information input screen 1001 includes an insured person information file input field 1002, an intervention information file input field 1003, a healthcare cost information file input field 1004, browse buttons 1005, and an analysis start button 1006. The insured person information file input field 1002, the intervention information file input field 1003, and the healthcare cost information file input field 1004 are input fields for inputting file names (paths) of a insured person information file, an intervention information file, and a healthcare cost information file, respectively. The browse button 1005 is a button operated to browse a file to be input. The analysis start button 1006 is a button operated to start the analysis after all the files are input.

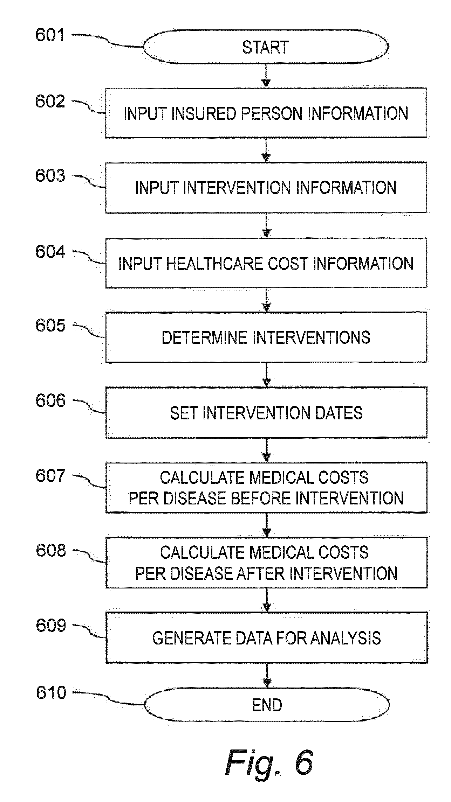

FIG. 6 is a flowchart of processing of generating the data for analysis based on the input insured person information, intervention information, and healthcare cost information.

When the healthcare service effect analysis apparatus 101 according to this embodiment starts data-for-analysis generation processing (601), the healthcare service effect analysis apparatus 101 displays the healthcare information input screen 1001 of FIG. 10 on the output unit 104.

In insured person information input step 602, the insured person information file name (path name) is input to the insured person information file input field 1002 by a user operating the browse button 1005. The healthcare service effect analysis apparatus 1001 acquires the insured person information from the input insured person information file. The acquired insured person information is managed by the insured person information management unit 120.

In Intervention information input step 603, the intervention information file name (path name) is input to the intervention information file input field 1003 by the user operating the browse button 1005. The healthcare service effect analysis apparatus 101 acquires the intervention information from the input intervention information file. The acquired intervention information is managed by the intervention information management unit 121.

In healthcare cost information input step 604, the healthcare cost information file name (path name) is input to the healthcare cost information file input field 1004 by the user operating the browse button 1005. The healthcare service effect analysis apparatus 101 acquires the healthcare cost information from the input healthcare cost information file. The acquired healthcare cost information is managed by the healthcare cost information management unit 122.

When the user inputs all the files, and operates the analysis start button 1006, Intervention determination step 605 is carried out.

In Intervention determination step 605, first, the intervention determination unit 110 acquires the insured person information managed by the insured person information management unit 120 and the intervention information managed by the intervention information management unit 121. Then, the intervention determination unit 110 collates the acquired insured person information and intervention information, thereby determining whether an intervention has been provided for each of the insured persons or not. Specifically, the intervention determination unit 110 collates the insured person ID 201 of the insured person information and the insured person ID 201 of the intervention information with each other, and assigns flags 502 to 504 representing absence/presence of the intervention service for the respective intervention services in the intervention information. For example, if an intervention service name A has been provided, the flag for the intervention service A is set to 1, and if the intervention service name A has not been provided, the flag for the intervention service A is set to 0. Moreover, if it is determined that an insured person has not been provided with the intervention service as a result of the collation between the insured person ID 201 of the insured person information and the insured person ID 201 of the intervention information, the nonintervention flag of the insured person is set to 1, and if the insured person has been provided with any of the intervention services, the nonintervention flag of the insured person is set to 0.

In Intervention setting step 606, first, the intervention date setting unit 111 acquires the insured person information managed by the insured person information management unit 120 and the intervention information managed by the intervention information management unit 121. Then, the intervention date setting unit 111 uses the intervention start date 303 of the acquired intervention information to set the intervention date 505 of the intervention groups and the nonintervention group. Specifically, the intervention start date 303 of the intervention information is set to the intervention date 505 of the intervention group. On the other hand, for the nonintervention group, the intervention date setting unit 111 calculates a probability distribution acquired intervention by normalizing a frequency distribution of the intervention start dates 303 of the intervention group, and randomly sets the intervention date 505 of the nonintervention group so that the calculated probability distribution of the intervention start date are equal to each other between the intervention group and the nonintervention group.

In Medical cost per disease before intervention calculation step 607, first, the medical cost per disease calculation unit 112 acquires the insured person information managed by the insured person information management unit 120 and the healthcare cost information managed by the healthcare cost information management unit 122. Then, the medical cost per disease calculation unit 112 uses the insured person ID 201 to collate the insured person information and the healthcare cost information with each other. Then, the medical cost per disease calculation unit 112 uses the intervention date set by the intervention date setting unit 111 and the clinical action month/year 403 in the healthcare cost information to extract healthcare costs having clinical action month and year before the intervention date, and uses the extracted healthcare costs to calculate the medical costs per disease before the intervention for each insured person. For example, an annual medical cost for one year before the intervention may be calculated as the medical cost per disease before the intervention.

In Medical cost per disease after intervention calculation step 608, first, the medical cost per disease calculation unit 112 acquires the insured person information managed by the insured person information management unit 120 and the healthcare cost information managed by the healthcare cost information management unit 122. Then, the medical cost per disease calculation unit 112 uses the insured person ID 201 to collate the insured person information and the healthcare cost information with each other. Then, the medical cost per disease calculation unit 112 uses the intervention date 505 set by the intervention date setting unit 111 and the clinical action month/year 403 in the healthcare cost information to extract healthcare costs having clinical action month and year after the intervention date, and uses the extracted healthcare costs to calculate an annual medical costs per disease after the intervention for each insured person. For example, an annual medical cost for one year or one to two years after the intervention may be calculated as the medical cost per disease after the intervention.

In Data-for-analysis generation step 609, the data-for-analysis generation unit 107 couples the intervention service flags 510 and the nonintervention flag 504 set by the intervention determination unit 110, the intervention date set by the intervention date setting unit 111, and the medical costs per disease before and after the intervention calculated by the medical cost per disease calculation unit 112 via the insured person ID 201, thereby generating the data for analysis of FIG. 5. The generated data for analysis is managed by the data-for-analysis management unit 123.

Then, the data-for-analysis generation processing is finished (610). Base data for analyzing the medical cost effect of the healthcare service is generated as a result of the processing.

A description is now given of processing of calculating the medical cost effects from the data for analysis.

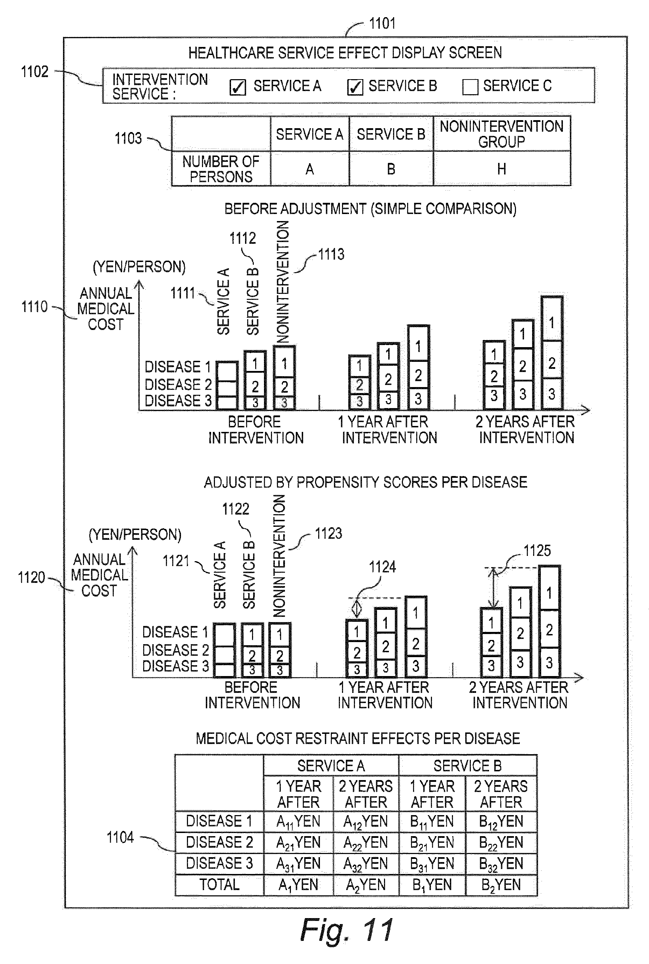

FIG. 11 is an explanatory diagram illustrating an example of a healthcare service effect display screen 1101 displayed on the output unit 104 by the healthcare service effect analysis apparatus 101.

The healthcare service effect display screen 1101 includes checkboxes 1102 for selecting the intervention services for which the medical cost effects are displayed, and an area 1103 for displaying the numbers of persons of the respective intervention services selected by the checkboxes 1102 and the number of persons in the nonintervention group.

Moreover, the healthcare service effect display screen 1101 includes a medical cost transition chart before adjustment 1110 for displaying the medical costs per disease before and after the interventions before the adjustment, and a medical cost transition chart after adjustment 1120 for displaying the medical costs per disease before and after the interventions adjusted by the propensity scores per disease. For example, the medical cost transition chart before adjustment 1110 includes medical costs per disease 1111 before and after the intervention of the service A, medical costs per disease 1112 before and after the intervention of the service B, and medical costs per disease 1113 before and after the intervention date of the nonintervention group. The medical cost transition chart after adjustment 1120 includes adjusted medical costs per disease 1121 before and after the intervention of the service A, adjusted medical costs per disease 1122 before and after the intervention of the service B, and adjusted medical costs per disease 1123 before and after the intervention date of the nonintervention group.

Moreover, the healthcare service effect display screen 1101 displays a medical cost restraint effect 1124 one year after the intervention and a medical cost restraint effect 1125 two years after the intervention with respect to the nonintervention group for the service A. Further, a lowest portion of the healthcare service effect display screen 1101 includes an area 1104 for displaying medical cost restraint effects per service and per disease.

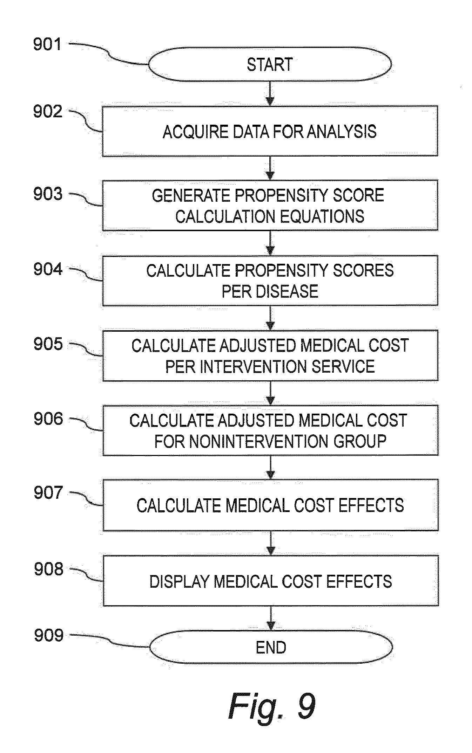

FIG. 9 is a flowchart of processing of calculating the medical cost effect from the data for analysis.

When the healthcare service effect analysis apparatus 101 according to this embodiment starts medical cost effect calculation processing (901), in Data-for-analysis acquisition step 902, the healthcare service effect analysis apparatus 101 acquires the data for analysis of FIG. 5 managed by the data-for-analysis management unit 123.

In Propensity score calculation equation generation step 903, the propensity score calculation equation generation unit 113 uses the acquired data for analysis to analyze each relationship between the medical cost per disease and the intervention service before the intervention and each relationship between the medical cost per disease and the nonintervention before the intervention, thereby generating an equation for calculating a propensity score $P$ for each of the intervention services and the nonintervention group. Specifically, the logistic regression analysis is carried out while each of the plurality of intervention flags and the nonintervention flag is considered as an objective variable, and the medical costs per disease before the intervention are considered as explanatory variables, thereby generating the equation for calculating the propensity score $P$ for each of the intervention services and the nonintervention group. In the example of FIG. 5, first, the logistic regression analysis is carried out while the intervention service A flag 502 is considered as the objective variable, and the medical costs per disease before intervention 511 are considered as the explanatory variables, thereby generating an equation for calculating a propensity score $P_A$ of the intervention service A. On this occasion, the propensity score $P_A$ of the intervention service A represents a probability of provision of the intervention service A calculated while the medical costs per disease before intervention are considered as conditions. When the regression coefficient of the medical cost per disease before intervention is set to β, the propensity score calculation equation is represented as Equation 1.

[Equation 1]

$$\frac{P_A}{1-P_A} = exp\big(\beta_{A1} medical\ cost\ for\ disease\ 1\ before\ intervention + \cdots$$

$$+ \beta_{AS} medical\ cost\ for\ disease\ S\ before\ intervention\big)$$

Similarly, the logistic regression analysis is carried out while the intervention service B flag 503 is considered as the objective variable, and the medical costs per disease before intervention 511 are considered as the explanatory variables, thereby generating an equation for calculating a propensity score $P_B$ of the intervention service B. On this occasion, the propensity score $P_B$ of the intervention service B represents a probability of provision of the intervention service B calculated while the medical costs per disease before intervention are considered as conditions.

This processing is repeated as many times as the number of the intervention services, thereby generating the equations for calculating the propensity scores for the respective services. Then, the logistic regression analysis is carried out while the nonintervention flag 504 is considered as the objective variable, and the medical costs per disease before intervention 511 are considered as the explanatory variables, thereby generating an equation for calculating a propensity score *Pnon-intervention* of the nonintervention group. On this occasion, the propensity score *Pnon-intervention* of the nonintervention represents a probability of non-provision of the intervention service calculated while the medical costs per disease before intervention are considered as conditions. The generated propensity score calculation equations are managed by the propensity score calculation equation management unit 124.

An adjustment can be made so that differences in the medical cost before the intervention among the plurality of intervention services, and between each of the plurality of intervention services and the nonintervention decrease by using the calculation equations.

In Propensity score per disease calculation step 904, the propensity score per disease calculation unit 114 decomposes each of the propensity score calculation equations generated by the propensity score calculation equation generation unit 113 into propensity score calculation equations for the respective diseases, thereby calculating propensity scores per disease. Specifically, the propensity score $P$ can be decomposed into a product of propensity scores per disease e as represented by Equation 2, and hence the propensity scores per disease e are calculated for the respective intervention services and the nonintervention group. The propensity score per disease e for each of the intervention service is represented by Equation 3. On this occasion, the propensity score per disease e for the intervention service represents a probability of the provision of the intervention service calculated while a medical cost of a certain disease s before the intervention is considered as a condition. Moreover, the propensity score per disease e is calculated for the nonintervention group. On this occasion, the propensity score per disease e for the nonintervention group represents a probability of the non-provision of the intervention service calculated while a medical cost of a certain disease s before the intervention is considered as a condition. The calculated propensity scores per disease e are managed by the propensity score per disease management unit 125.

[Equation 2]

$$\frac{P}{1-P} = \frac{e_{disease\ 1}}{1-e_{disease\ 1}} \frac{e_{disease\ 2}}{1-e_{disease\ 2}} \cdots \frac{e_{disease\ s}}{1-e_{disease\ s}}$$

[Equation 3]

$$\frac{e_{disease\,s}}{1 - e_{disease\,s}} = \exp\left(\beta_S\, medical\ cost\ for\ disease\ S\ before\ intervention\right)$$

In Adjusted medical cost per intervention service calculation step 905, first, the adjusted medical cost calculation unit 115 acquires the propensity scores per disease for the respective intervention services managed by the propensity score per disease management unit 125. Then, the adjusted medical cost calculation unit 115 calculates the adjusted medical costs per disease adjusted by weighting the medical costs per disease before and after the intervention by the acquired propensity score per disease for each of the intervention services. Specifically, when the insured persons are indexed by $i$=1 to $N$, and the intervention service flag 510 is represented as $Z_i$, the adjusted medical costs per disease before and after the intervention are calculated by using Equation 4.

[Equation 4]

$$\frac{1}{N} \sum_{i=1}^{N} \frac{disease\ S\ medical\ cost_i Z_i}{e_{disease\,s,i}}$$

This processing is repeated as many number of times as the number of the intervention services, thereby calculating the adjusted medical costs per disease before and after the intervention for each of the intervention services.

In Adjusted medical cost for nonintervention group calculation step 906, first, the adjusted medical cost calculation unit 115 acquires the propensity scores per disease for the nonintervention group managed by the propensity score per disease management unit 125. Then, the adjusted medical cost calculation unit 115 calculates the adjusted medical costs per disease adjusted by weighting the medical costs per disease before and after the intervention by the acquired propensity score per disease for the nonintervention group. Specifically, when the insured persons are indexed by i=1 to $N$, and the nonintervention flag 504 is represented as $Z_i$, the adjusted medical costs per disease before and after the intervention date of the nonintervention group are calculated by using Equation 4.

The processing in Steps 905 and 906 can make the adjustment so that differences in the medical cost per disease before the intervention among the plurality of the intervention services and the nonintervention group decrease, and the medical costs per disease between each of the plurality of intervention services and the nonintervention group after the intervention can be compared with each other.

In Medical cost effect calculation step 907, the medical cost effect calculation unit 116 subtracts the adjusted medical cost per disease after the intervention of the intervention service from the adjusted medical cost per disease after the intervention of the nonintervention group calculated by the adjusted medical cost calculation unit 115, thereby calculating the medical cost restraint effect for each of the intervention services.

In Medical cost effect display step 908, the effect analysis unit 108 generates data for displaying, on the output unit 104, the healthcare service effect display screen 1101 including the adjusted medical costs per disease for the respective intervention services and the nonintervention group calculated by the adjusted medical cost calculation unit 115, and the medical cost restraint effects for the respective intervention services calculated by the medical cost effect calculation unit 116. Specifically, when the user selects an intervention service to be displayed by using the intervention service selection checkbox 1102, the medical costs before the adjustment before and after the intervention of the selected intervention service, the medical costs adjusted by the propensity score per disease, and the medical cost restraint effects are displayed.

Then, the calculation processing for the medical cost restraint effect is finished (909).

As described above, the healthcare service effect analysis system according to this invention can calculate the adjusted medical costs per disease for the plurality of intervention services and the nonintervention group acquired by weighting the medical costs per disease before and after the intervention by using the propensity scores per disease calculated from the medical costs per disease before the intervention. As a result, the adjustment can be made so that the differences in the medical cost per disease before the intervention among the plurality of the intervention services and the nonintervention group decrease, and the medical costs per disease between each of the plurality of intervention services and the nonintervention group after the intervention can thus be compared with each other. As a result, the medical cost restraint effect by the healthcare service can be accurately analyzed and displayed. Moreover, the medical cost restraint effect per disease can be accurately analyzed and displayed, and diseases high in the medical cost restraint effects can be analyzed and displayed.

Moreover, in the embodiment described above, a description has been given of the sequence of processing of generating the data for analysis from the insured person information, the intervention information, and the healthcare cost information, and further calculating the medical cost effects from the generated data for analysis, but data for analysis which has already been generated may be input, and the medical cost effects may be calculated from the input data for analysis.

Moreover, in the embodiment described above, a description has been given of such an example that the propensity score per disease calculation unit 114 calculates and uses the propensity scores per disease to calculate the adjusted medical costs before and after the intervention, but the processing in Propensity score per disease calculation step 904 of FIG. 9 may be omitted, and the propensity scores $P$ of FIG. 7 generated by the propensity score calculation equation generation unit 113 may be used to calculate the adjusted medical costs before and after the interventions.

Specifically, first, the insured persons are indexed by $i$=1 to $N$, the intervention service flag 510 shown in FIG. 5 is represented as $Zi$, and an adjusted medical cost for all the diseases is calculated from the medical costs for all the diseases (sum for the diseases 1 to $S$) before and after the intervention date of the intervention group are calculated by using Equation 5. This processing is repeated as many number of times as the number of the intervention services, thereby calculating the adjusted medical costs for all the diseases before and after the intervention for each of the intervention services. Then, the insured persons are indexed by $i$=1 to $N$, the nonintervention flag 504 shown in FIG. 5 is represented as $Zi$, and the adjusted medical cost for all the diseases before and after the intervention date for the nonintervention group is calculated by using Equation 5. As a result, the adjusted medical costs before and after the interventions can be calculated.

[Equation 5]

$$\frac{1}{N} \sum_{i=1}^{N} \frac{medical\ cost\ for\ all\ diseases\ _{i}Z_{i}}{P_{i}}$$

The method described above can be applied to a case where a difference in an average medical cost before the interventions between the intervention group and the nonintervention group is equal for any diseases. In this case, the processing in Propensity score per disease calculation step (904) of FIG. 9 can be omitted, and the processing can decrease.

Moreover, in the embodiment described above, the difference in the average medical cost before the intervention between each of the intervention groups and the nonintervention group may be calculated for each of the diseases, and the adjusted medical cost may be calculated by considering the sign of the calculated difference in the embodiment described before.

In this case, the effect analysis unit 108 of FIG. 1 includes the medical cost difference per disease calculation unit 130 and the disease extraction unit 131. The medical cost difference per disease calculation unit 130 calculates a difference in the average medical cost between each of the intervention groups of the intervention service and the nonintervention group for each of the diseases. The disease extraction unit 131 determines the sign of the medical cost difference per disease calculated by the medical cost difference per disease calculation unit 130, and extracts diseases same in the sign of the difference.

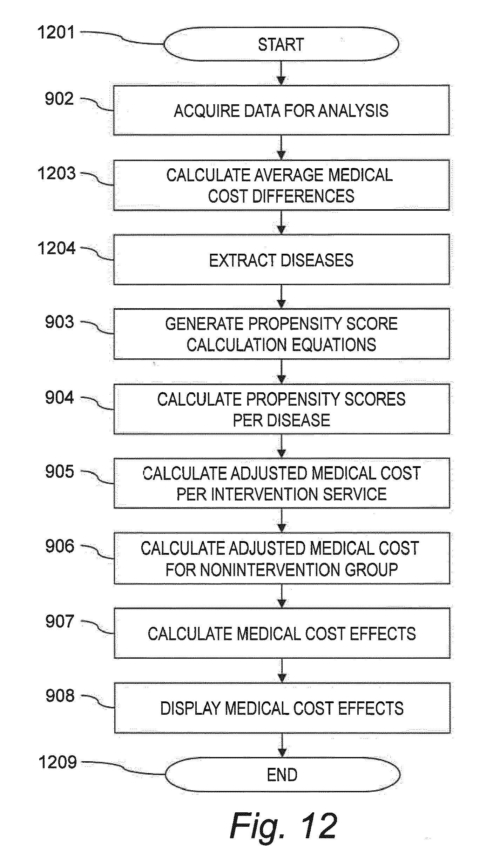

FIG. 12 is a flowchart of the adjusted medical cost calculation processing considering the average medical cost difference between each of the intervention groups and the nonintervention group. A description is given of points different from the medical cost effect calculation processing of FIG. 9, and a description is not given of the same points.

When the adjusted medical cost calculation processing is started (1201), first, the healthcare service effect analysis apparatus 101 carries out processing in Data-for-analysis acquisition step 902. In Data-for-analysis acquisition step 902, the healthcare service effect analysis apparatus 101 acquires the data for analysis of FIG. 5 managed by the data-for analysis management unit 123.

In Average medical cost difference calculation step 1203, the medical cost difference per disease calculation unit 130 calculates the average medical cost for each of the diseases and for each of the intervention service groups and the nonintervention group, and calculates an average medical cost difference per disease, which is a difference between the average medical cost for each of the diseases in the intervention group for each of the intervention services and the average medical cost for each of the diseases of the nonintervention group.

In Disease extraction step 1204, the disease extraction unit 131 determines the sign of the difference in the average medical cost per disease between each of the intervention services and the nonintervention group for each of the diseases calculated by the medical cost per disease difference calculation unit 130. Then, the disease extraction unit 131 extracts positive sign difference diseases positive in sign and negative sign difference diseases negative in sign.

In Propensity score calculation equation generation step 903, the propensity score calculation equation generation unit 113 generates an equation for calculating a propensity score $P$ for each of the positive and negative sign difference diseases extracted by the disease extraction units 131 and for each of the intervention services and the nonintervention group. Specifically, first, the logistic regression analysis is carried out while each of the plurality of intervention service flags and the nonintervention flag is considered as an objective variable, and the medical costs before the intervention for the positive sign difference diseases extracted by the disease extraction unit 131 are considered as explanatory variables, thereby generating the equation for calculating the propensity score $P$ (positive sign difference disease) for each of the intervention services and the nonintervention group. Then, the logistic regression analysis is carried out while each of the plurality of intervention service flags and the nonintervention flag is considered as an objective variable, and the medical costs before the intervention for the negative sign difference diseases extracted by the disease extraction unit 131 are considered as explanatory variables, thereby generating the equation for calculating the propensity score $P$ (negative sign difference disease) for each of the intervention services and the nonintervention group.

In Propensity score per disease calculation step 904, the propensity score per disease calculation unit 114 decomposes the propensity score calculation equations for the positive sign difference diseases and the propensity score calculation equations for the negative sign difference diseases generated by the propensity score calculation equation generation unit 113 into propensity score calculation equations for the respective diseases, thereby calculating propensity scores per disease for the positive sign difference diseases and the negative sign difference diseases.

In Adjusted medical cost per intervention service calculation step 905 and Adjusted medical cost for nonintervention group calculation step 906, the adjusted medical cost calculation unit 115 uses the propensity scores per disease for the positive sign difference diseases and the negative sign difference diseases calculated in Propensity score per disease calculation step 904 to carry out the same processing as the processing described above, thereby calculating the adjusted medical costs. Specifically, first, the adjusted medical cost calculation unit 115 uses the propensity scores per disease for the positive sign difference diseases to calculate the adjusted medical costs per disease for the positive sign difference diseases, and uses the propensity scores per disease for the negative sign difference diseases to calculate the adjusted medical costs per diseases for the negative sign difference diseases.

In Medical cost effect calculation step 907 and Medical cost effect display step 908, the same processing as the processing described above is carried out, thereby calculating the medical cost restraint effect for each of the intervention services from adjusted medical costs per disease for the positive sign difference diseases and the negative sign difference diseases of each of the intervention service groups and the nonintervention group, and the calculated medical cost restraint effects are displayed.

Then, the calculation processing for the medical cost restraint effects is finished (1209).

As described above, the propensity scores per disease are calculated by considering the sign of the difference in the average medical cost before intervention between each of the intervention groups and the nonintervention group, thereby calculating adjusted medical costs before and after the interventions. Therefore, even if the signs of the difference in the average medical cost before the intervention are different from each other between each of the intervention groups and the nonintervention group, the difference in the average medical cost before intervention can be reduced, and the medical cost restraint effect by the intervention service can be more accurately analyzed.

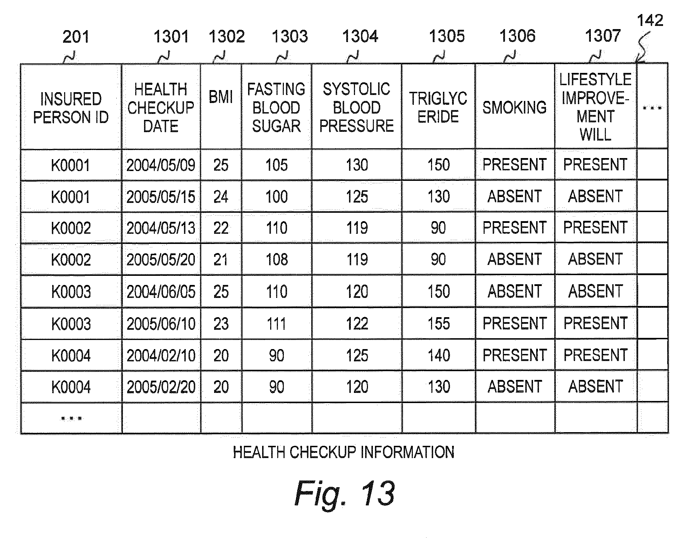

In the embodiment described above, a description has been given of such an example that the adjusted medical cost is calculated by using the medical cost per disease before intervention, but the adjusted medical cost may be calculated by using the health checkup information shown in FIG. 13 such as the BMI, the blood sugar level, the blood pressure, the lipid, and a medical inquiry about the lifestyle.

In this case, the effect analysis unit 108 of FIG. 1 includes the health checkup item contribution calculation unit 140 and the contributed disease determination unit 141, and the database 106 stores the health checkup information management unit 142 for managing the health checkup information. The health checkup item contribution calculation unit 140 calculates a contribution of the health checkup information to the medical cost per disease. The contributed disease determination unit 141 determines diseases to which health checkup items contribute.

FIG. 13 is an explanatory diagram illustrating an example of the health checkup information managed by the health checkup information management unit 142.

The health checkup information management unit 142 stores information acquired by the medical inquiry and tests, and includes data such as insured person IDs 201, health checkup dates 1301, BMIs 1302, fasting blood sugars 1303, systolic blood pressures 1304, triglycerides 1305, smoking 1306, and lifestyle improvement wills 1307.

The insured person ID 201 is identification information for identifying a health insurance insured person. The health checkup date 1301 is a date when the insured person took the health checkup. The BMI 1302, the fasting blood sugar 1303, the systolic blood pressure 1304, and the triglyceride 1305 are results of the tests provided for the insured person. The smoking 1306 and the lifestyle improvement will 1307 are results of the medical inquiry provided for the insured person, and are information on presence/absence of smoking and information on presence/absence of lifestyle improvement will. It should be noted that the health checkup information management unit 142 may store data such as sex and age other than the shown data.

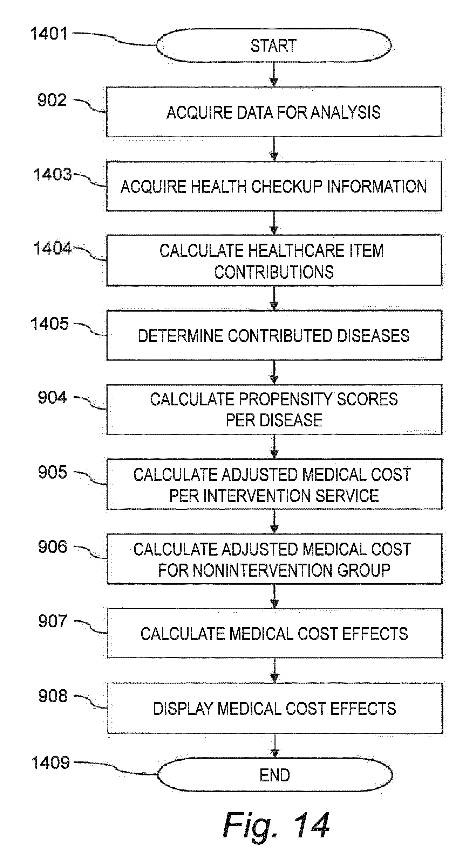

FIG. 14 is a flowchart of the adjusted medical cost calculation processing considering the health checkup information. A description is given of points different from the medical cost effect calculation processing of FIG. 9, and a description is not given of the same points.

When the adjusted medical cost calculation processing is started (1401), first, the healthcare service effect analysis apparatus 101 carries out processing in Data-for-analysis acquisition step 902. In Data-for-analysis acquisition step 902, the healthcare service effect analysis apparatus 101 acquires the data for analysis of FIG. 5 managed by the data-for analysis management unit 123.

In health checkup information acquisition step 1403, the healthcare service effect analysis apparatus 101 acquires the health checkup information of FIG. 13 managed by the health checkup information management unit 142.

In Health checkup item contribution calculation step 1404, first, the health checkup item contribution calculation unit 140 uses the insured person ID 201 to collate the acquired data for analysis and the health checkup information with each other. Then, the health checkup item contribution calculation unit 140 compares the intervention date 505 of the data for analysis and the health checkup date 1301 of the health checkup information with each other, thereby extracting health checkup information recording a health checkup date 1301 before the intervention date 505 (health checkup information before the intervention) for each of the insured person IDs 201. Then, the health checkup item contribution calculation unit 140 uses the extracted health checkup information before the intervention and the data for analysis to apply the regression analysis to a relationship between the health checkup item before the intervention and the medical cost per disease, thereby calculating a regression coefficient representing a contribution of the health checkup item to the medical cost per disease, and a significance probability thereof. This processing is carried out for each of the health checkup items and each of the diseases.

In Contributed disease determination step 1405, the contributed disease determination unit 141 determines health checkup items 1 to $K$ contributing a certain disease $S$ from significance probabilities of the regression coefficients of the health checkup items calculated by the health checkup item contribution calculation unit 140. Specifically, when the significance probability of the regression coefficient is less than 5%, the health checkup item thereof is determined to contribute to the certain disease $S$. This processing is carried out for each of the health checkup items and each of the diseases.

In Propensity score per disease calculation step 904, the propensity score per disease calculation unit 114 uses the information on the health checkup items determined to contribute to the disease by the contributed disease determination unit 141 to calculate a propensity score per disease by adding information on the health checkup items to the propensity score per disease of the contributed disease. Specifically, if $K$ health checkup items contributing to the certain disease S exist, the propensity score per disease is calculated by using Equation 6. On this occasion, $\gamma$ is a regression coefficient for each of the health checkup items.

[Equation 6]

$$\frac{e_{disease\,s}}{1-e_{disease\,s}} = exp\big(\beta_S\,medical\;cost\;for\;disease\;S\;before\;intervention$$

$$+\,\gamma_1 health\;checkup\;item\;1 + \cdots + \gamma_K health\;checkup\;item\;K\big)$$

In Adjusted medical cost per intervention service calculation step 905, Adjusted medical cost for nonintervention group calculation step 906, Medical cost effect calculation step 907, and Medical cost effect display step 908, the same processing as the processing described above is carried out by using the propensity scores per disease to which the health checkup information calculated by the propensity score per disease calculation unit 114 is added. Specifically, an adjusted medical cost for each of the intervention service groups and the nonintervention group are calculated by using the propensity score per disease to which the health checkup information is added, the medical cost restraint effect is calculated for each of the intervention services, and the calculated medical cost restraint effects are displayed.

Then, the calculation processing for the medical cost restraint effect is finished (1409).

As described above, the adjusted medical costs before and after the intervention can be calculated by considering not only the medical cost per disease before the intervention but also the test values, life styles, improvement wills, the sex, and the age before the intervention contributing to the disease, and hence the medical cost restraint effect by the intervention service can be more accurately analyzed.

Such an example that the logistic regression analysis is used to calculate the propensity scores $P$ and the propensity scores per disease $e$ has been described in the embodiment described above, but other analysis methods may be used. For example, analysis methods such as the Probit regression analysis, the discrimination analysis, the decision tree, the neural network, the generalized additive model, and the multinomial logit model may be used for the calculation. Such a conditional probability $pr$((intervention service) | (medical cost per disease before intervention)) that the intervention service is provided and such a conditional probability $pr$((nonintervention group) | (medical cost per disease before intervention)) that the intervention service is not provided while the medical cost per disease before the intervention is considered as a condition may be calculated as the propensity score $P$ by using these methods. Moreover, such a conditional probability $pr$((intervention service) | (medical cost for a certain disease $s$ before intervention)) that the intervention service is provided and a conditional probability $pr$((nonintervention group) | (medical cost for the disease $s$ before intervention)) that the intervention service is not provided while the medical cost for the certain disease $s$ before intervention is considered as a condition may be calculated as the propensity score per disease $e$ by using these methods.

A description has been given of the example of calculating the medical cost effect in the embodiment described above, but a restraint effect on the number of healthcare costs and the number of dates of the clinical action may be calculated by the same processing.

This invention is not limited to the above-described embodiments but includes various modifications. The above-described embodiments are explained in details for better understanding of this invention and are not limited to those including all the configurations described above. A part of the configuration of one embodiment may be replaced with that of another embodiment; the configuration of one embodiment may be incorporated to the configuration of another embodiment. A part of the configuration of each embodiment may be added, deleted, or replaced by that of a different configuration.

The above-described configurations, functions, processing modules, and processing means, for all or a part of them, may be implemented by hardware: for example, by designing an integrated circuit. The above-described configurations and functions may be implemented by software, which means that a processor interprets and executes programs providing the functions. The information of programs, tables, and files to implement the functions may be stored in a storage device such as a memory, a hard disk drive, or an SSD (a Solid State Drive), or a storage medium such as an IC card, or an SD card. The drawings shows control lines and information lines as considered necessary for explanation but do not show all control lines or information lines in the products. It can be considered that almost of all components are actually interconnected.

**Claims**

1. An analysis system for executing a program to analyze an effect of a healthcare guidance business, comprising:

   a processor for executing the program;
   a memory for storing the program;
   an input unit configured to control the processor to receive a medical cost of a insured person, intervention information on a provision of an intervention service and a start date of the intervention service;
   a propensity score calculation unit configured to control the processor to analyze a relationship between the medical cost before the provision of the intervention service and the intervention information, and to calculate a propensity score of an intervention group representing that the intervention service is provided and a propensity score of a nonintervention group representing that the intervention service is not provided from the medical cost before the provision of the intervention service; and
   an adjusted medical cost calculation unit configured to control the processor to calculate adjusted medical costs of the intervention group before and after the provision of the intervention service by using the propensity score of the intervention group and medical costs of the intervention group before and after the provision of the intervention service, and to calculate adjusted medical costs of the nonintervention group before and after the provision of the intervention service by using the propensity score of the nonintervention group and medical costs of the nonintervention group before and after the provision of the intervention service.

2. The analysis system according to claim 1, wherein:

the input unit inputs a medical cost for each disease of the insured person;

the propensity score calculation unit is configured to analyze a relationship between a medical cost for each disease before the provision of the intervention service and the intervention information for the each disease, and to calculate a propensity score for the each disease of the intervention group representing that the intervention service is provided for the each disease and a propensity score of the nonintervention group representing that the intervention service is not provided from the medical cost for the each disease before the provision of the intervention service, for the each disease; and

the adjusted medical cost calculation unit is configured to calculates adjusted medical costs of the each disease of the intervention group before and after the provision of the intervention service by multiplying the medical costs for the each disease of the intervention group before and after the provision of the intervention service by a reciprocal of the propensity score for the each disease of the intervention group to, and to calculate adjusted medical costs for the each disease of the nonintervention group before and after the provision of the intervention service by multiplying the medical costs of the each disease of the nonintervention group before and after the provision of the intervention service by a reciprocal of the propensity score for the each disease of the nonintervention group.

3. The analysis system according to claim 1, further comprising:

an intervention determination unit configured to control the processor to classify the insured persons into the intervention group where the intervention service is provided and the nonintervention group where the intervention service is not provided by using the intervention information;

an intervention date setting unit configured to control the processor to set an intervention date for the insured person of the intervention group based on the start date of the intervention service, and to set an intervention date for the insured person of the nonintervention group based on a distribution of the set intervention date for the insured person of the intervention group; and

a medical cost calculation unit configured to control the processor to divide the medical cost into medical costs before and after the provision of the intervention service based on the intervention date, and to calculate the medical costs before and after the provision of the intervention service.

4. The analysis system according to claim 1, further comprising a medical cost effect calculation unit configured to control the processor to calculate a medical cost restraint effect by the intervention service by using the adjusted medical cost after the provision of the intervention service of the intervention group and the adjusted medical cost after the provision of the intervention service of the nonintervention group.

5. The analysis system according to claim 4, wherein the analysis system is configured to generate data for displaying the adjusted medical costs before and after the provision of the intervention service of the intervention group, the adjusted medical costs before and after the provision of the intervention service of the nonintervention group and the medical cost restraining effect.

6. The analysis system according to claim 2, further comprising:

a medical cost difference per disease calculation unit configured to control the processor to calculate a difference between an average medical cost for the each disease of the intervention group and an average medical cost for the each disease of the nonintervention group; and

a disease extraction unit configured to control the processor to determine a sign of the calculated difference in the average medical cost for the each disease between the average medical costs, and to extract the diseases same in the sign of the difference, wherein:

the propensity score calculation unit configured to control the processor to analyze the sign of the difference for the each disease, and to calculate a propensity score of a disease positive in the difference and a propensity score of a disease negative in the difference; and

the adjusted medical cost calculation unit is configured to:

calculate adjusted medical costs for the disease positive in the difference before and after the provision of the intervention service by multiplying the medical costs for the each disease before and after the provision of the intervention service by a reciprocal of the propensity score of the disease positive in the difference; and

calculate adjusted medical costs for the disease negative in the difference before and after the provision of the intervention service by multiplying the medical costs for the each disease before and after the provision of the intervention service by a reciprocal of the propensity score of the disease negative in the difference.

7. The analysis system according to claim 2, wherein:

the input unit is configured to input information on a health checkup of the insured person;
the analysis system further comprises:

a health checkup item contribution calculation unit configured to control the processor to calculate a contribution of the information on the health checkup to the medical cost of the each disease; and
a contributed disease determination unit configured to control the processor to determine a disease contributed by the information on the health checkup; and
the propensity score calculation unit is configured to calculate a propensity score for the each disease by using the information on the health checkup contributing to the disease.

8. An analysis method for analyzing an effect of a healthcare guidance business by using a computer, the computer including a processor for executing a program and a memory for storing the program, and being configured to execute the program,
the analysis method including:

an input step of receiving, by the processor, a medical cost of an insured person, intervention information on a provision of an intervention service and a start date of the intervention service;
a propensity score calculation step of analyzing, by the processor, a relationship between the medical cost before the provision of the intervention service and the intervention information, and calculating, by the processor, a propensity score of an intervention group representing that the intervention service is provided and a propensity score of a nonintervention group representing that the intervention service is not provided from the medical cost before the provision of the intervention service; and
an adjusted medical cost calculation step of calculating, by the processor, adjusted medical costs of the intervention group before and after the provision of the intervention service by using the propensity score of the intervention group and medical costs of the intervention group before and after the provision of the intervention service, and calculating, by the processor, adjusted medical costs of the nonintervention group before and after the provision of the intervention service by using the propensity score of the nonintervention group and medical costs of the nonintervention group before and after the provision of the intervention service.

9. The analysis method according to claim 8, wherein:

the input step includes step of inputting a medical cost for each disease of the insured person;
the propensity score calculation step includes analyzing a relationship between a medical cost for each disease before the provision of the intervention service and the intervention information for the each disease, and calculating a propensity score for the each disease of the intervention group representing that the intervention service is provided for the each disease and a propensity score of the nonintervention group representing that the intervention service is not provided from the medical cost for the each disease before the provision of the intervention service, for the each disease; and
the adjusted medical cost calculation step includes calculating adjusted medical costs for the each disease of the intervention group before and after the provision of the intervention service by multiplying the medical costs for the each disease of the intervention group before and after the provision of the intervention service by a reciprocal of the propensity score for the each disease of the intervention group, and calculating adjusted medical costs for the each disease of the nonintervention group before and after the provision of the intervention service by multiplying the medical costs for the each disease of the nonintervention group before and after the provision of the intervention service by a reciprocal of the propensity score for the each disease of the nonintervention group.

10. The analysis method according to claim 8, further including:

an intervention determination step of classifying, by the processor, the insured persons into the intervention group where the intervention service is provided and the nonintervention group where the intervention service is not provided by using the intervention information;
an intervention date setting step of setting, by the processor, an intervention date for the insured person of the intervention group based on the start date of the intervention service, and setting, by the processor, an intervention date for the insured person of the nonintervention group based on a distribution of the set intervention date for the insured person of the intervention group; and

a medical cost calculation step of dividing, by the processor, the medical cost into medical costs before and after the provision of the intervention service based on the intervention date, and calculating, by the processor, the medical costs before and after the provision of the intervention service.

11. The analysis method according to claim 8, further including a medical cost effect calculation step of calculating, by the processor, a medical cost restraint effect by the intervention service by using the adjusted medical cost after the provision of the intervention service of the intervention group and the adjusted medical cost after the provision of the intervention service of the nonintervention group.

12. The analysis method according to claim 11, further including a step of generating data for displaying the adjusted medical costs before and after the provision of the intervention service of the intervention group, the adjusted medical costs before and after the provision of the intervention service of the nonintervention group and the medical cost restraining effect.

13. The analysis method according to claim 9, further including:

a medical cost difference per disease calculation step of calculating, by the processor, a difference between an average medical cost for the each disease of the intervention group and an average medical cost for the each disease of the nonintervention group; and

a disease extraction step of determining, by the processor, a sign of the calculated difference in the average medical cost for the each disease between the average medical costs, and extracting, by the processor, the diseases same in the sign of the difference, wherein:

the propensity score calculation step includes analyzing the sign of the difference for the each disease, and calculating a propensity score of a disease positive in the difference and a propensity score of a disease negative in the difference; and

the adjusted medical cost calculation step includes:

calculating adjusted medical costs for the disease positive in the difference before and after the provision of the intervention service by multiplying the medical costs for the each disease before and after the provision of the intervention service by a reciprocal of the propensity score of the disease positive in the difference; and

calculating adjusted medical costs for the disease negative in the difference before and after the provision of the intervention service by multiplying the medical costs for the each disease before and after the provision of the intervention service by a reciprocal of the propensity score of the disease negative in the difference.

14. The analysis method according to claim 9, wherein:

the input step including inputting information on a health checkup of the insured person;

the analysis method further includes:

a health checkup item contribution calculation step of calculating, by the processor, a contribution of the information on the health checkup to the medical cost of the each disease; and

a contributed disease determination unit for determining, by the processor, a disease contributed by the information on the health checkup; and

the propensity score calculation step including calculating a propensity score for the each disease by using the information on the health checkup contributing to the disease.

*Fig. 1*

| | | | | | 120 |
|---|---|---|---|---|---|
| 201 | 202 | 203 | 204 | 205 | |
| INSURED PERSON ID | START DATE | TERMINATION DATE | SEX | BIRTHDATE | ... |
| K0001 | 2003/04/01 | | MALE | 1978/10/25 | |
| K0002 | 1978/06/05 | | MALE | 1960/06/20 | |
| K0004 | 1960/06/05 | 2011/06/10 | FEMALE | 1950/07/20 | |
| ... | | | | | |

INSURED PERSON INFORMATION

*Fig. 2*

| | | | 121 |
|---|---|---|---|
| 201 | 302 | 303 | |
| INSURED PERSON ID | INTERVENTION SERVICE NAME | INTERVENTION START DATE | ... |
| K0001 | SERVICE A | 2004/06/05 | |
| K0002 | SERVICE B | 2004/06/10 | |
| K0004 | SERVICE B | 2004/07/10 | |
| ... | | | |

INTERVENTION INFORMATION

*Fig. 3*

| SEARCH NUMBER | INSURED PERSON ID | CONSULTATION MONTH/YEAR | DISEASE NAME | MEDICAL COST | ... |
|---|---|---|---|---|---|
| 401 | 201 | 403 | 404 | 405 | 122 |
| 11 | K0001 | 2004/06 | DISEASE 1 | GT11 | |
| 12 | K0001 | 2004/07 | DISEASE 1 | GT12 | |
| 13 | K0001 | 2004/08 | DISEASE 2 | GT13 | |
| 21 | K0002 | 2004/04 | DISEASE 2 | GT21 | |
| 22 | K0002 | 2004/09 | DISEASE 2 | GT22 | |
| 31 | K0003 | 2004/06 | DISEASE 3 | GT31 | |
| 32 | K0003 | 2004/12 | DISEASE 3 | GT32 | |
| 41 | K0004 | 2005/01 | DISEASE 3 | GT41 | |
| ... | | | | | |

HEALTHCARE COST INFORMATION

Fig. 4

510

123

| INSURED PERSON ID 201 | INTERVENTION SERVICE A FLAG 502 | INTERVENTION SERVICE B FLAG 503 | ... | NONINTERVENTION FLAG 504 | INTERVENTION DATE 505 |
|---|---|---|---|---|---|
| K0001 | 1 | 0 | | 0 | 2008/09/01 |
| K0002 | 0 | 1 | | 0 | 2008/10/01 |
| K0003 | 0 | 0 | | 1 | 2008/09/01 |
| ... | | | | | |

| INSURED PERSON ID 201 | 511 | | | 512 | | | 513 | | |
|---|---|---|---|---|---|---|---|---|---|
| | MEDICAL COST | | | | | | | | |
| | DISEASE 1 BEFORE INTERVENTION | ... | DISEASE S BEFORE INTERVENTION | ALL DISEASES ONE YEAR AFTER INTERVENTION | DISEASE 1 ONE YEAR AFTER INTERVENTION | ... | DISEASE S ONE YEAR AFTER INTERVENTION | ALL DISEASES TWO YEARS AFTER INTERVENTION | ... |
| K0001 | | | | | | | | | |
| K0002 | | | | | | | | | |
| K0003 | | | | | | | | | |
| ... | | | | | | | | | |

DATA FOR ANALYSIS

Fig. 5

601 — START

602 — INPUT INSURED PERSON INFORMATION

603 — INPUT INTERVENTION INFORMATION

604 — INPUT HEALTHCARE COST INFORMATION

605 — DETERMINE INTERVENTIONS

606 — SET INTERVENTION DATES

607 — CALCULATE MEDICAL COSTS
PER DISEASE BEFORE INTERVENTION

608 — CALCULATE MEDICAL COSTS
PER DISEASE AFTER INTERVENTION

609 — GENERATE DATA FOR ANALYSIS

610 — END

*Fig. 6*

302          702          124

| INTERVENTION SERVICE | PROPENSITY SCORE P CALCULATION EQUATION |
|---|---|
| SERVICE A | $\dfrac{P_A}{1-P_A} = exp(\beta_{AI}\,disease1\ medical\ cost\ befor\ intervention + \cdots$ $+ \beta_{AS}\,diseaseS\ medical\ cost\ befor\ intervention )$ |
| SERVICE B | $\dfrac{P_B}{1-P_B} = exp(\beta_{BI}\,disease1\ medical\ cost\ befor\ intervention + \cdots$ $+ \beta_{BS}\,diseaseS\ medical\ cost\ befor\ intervention )$ |
| ... | |
| NONINTERVENTION GROUP | $\dfrac{P_{nonintervention}}{1-P_{nonintervention}}$ $= exp(\beta_{non1}\,disease1\ medical\ cost\ befor\ intervention + \cdots$ $+ \beta_{nonS}\,disease1\ medical\ cost\ befor\ intervention )$ |

Fig. 7

302      404            803      125

| INTERVENTION SERVICE | DISEASE NAME | PROPENSITY SCORE PER DISEASE e |
|---|---|---|
| SERVICE A | DISEASE 1 | $\dfrac{e_{A\,disease1}}{1-e_{A\,disease1}} = exp(\ \beta_{A1} disease1\ medical\ cost\ before\ intervention\ )$ |
| SERVICE A | DISEASE 2 | $\dfrac{e_{A\,disease2}}{1-e_{A\,disease2}} = exp(\ \beta_{A2} disease2\ medical\ cost\ before\ intervention\ )$ |
| ... | | |
| SERVICE B | DISEASE 1 | $\dfrac{e_{B\,disease1}}{1-e_{B\,disease1}} = exp(\ \beta_{B1} disease1\ medical\ cost\ before\ intervention\ )$ |
| SERVICE B | DISEASE 2 | $\dfrac{e_{B\,disease2}}{1-e_{B\,disease2}} = exp(\ \beta_{B2} disease2\ medical\ cost\ before\ intervention\ )$ |
| ... | | |
| NON-INTERVENTION GROUP | DISEASE 1 | $\dfrac{e_{nonintervention\,disease1}}{1-e_{nonintervention\,disease1}} = exp(\ \beta_{nonintervention1} disease1\ medical\ cost\ before\ intervention )$ |
| NON-INTERVENTION GROUP | DISEASE 2 | $\dfrac{e_{nonintervention\,disease2}}{1-e_{nonintervention\,disease2}} = exp(\ \beta_{nonintervention2} disease2\ medical\ cost\ before\ intervention )$ |

PROPENSITY SCORE PER DISEASE      *Fig. 8*

901 — START

902 — ACQUIRE DATA FOR ANALYSIS

903 — GENERATE PROPENSITY SCORE
CALCULATION EQUATIONS

904 — CALCULATE PROPENSITY SCORES
PER DISEASE

905 — CALCULATE ADJUSTED MEDICAL COST
PER INTERVENTION SERVICE

906 — CALCULATE ADJUSTED MEDICAL COST
FOR NONINTERVENTION GROUP

907 — CALCULATE MEDICAL COST EFFECTS

908 — DISPLAY MEDICAL COST EFFECTS

909 — END

*Fig. 9*

1001

**HEALTH INSURANCE INFORMATION INPUT SCREEN**

■ INSURED PERSON INFORMATION

1002    1005

| INSURED PERSON INFORMATION FILE | BROWSE |

■ INTERVENTION INFORMATION

1003    1005

| INTERVENTION INFORMATION FILE | BROWSE |

■ HEALTHCARE COST INFORMATION

1004    1005

| HEALTHCARE COST INFORMATION FILE | BROWSE |

1006

| START ANALYSIS |

*Fig. 10*

1101

## HEALTHCARE SERVICE EFFECT DISPLAY SCREEN

1102

INTERVENTION SERVICE : ☑ SERVICE A   ☑ SERVICE B   ☐ SERVICE C

1103

| | SERVICE A | SERVICE B | NONINTERVENTION GROUP |
|---|---|---|---|
| NUMBER OF PERSONS | A | B | H |

BEFORE ADJUSTMENT (SIMPLE COMPARISON)

1110

(YEN/PERSON)

ANNUAL MEDICAL COST

1112
1113
1111

SERVICE A
SERVICE B
NONINTERVENTION

DISEASE 1
DISEASE 2
DISEASE 3

BEFORE INTERVENTION        1 YEAR AFTER INTERVENTION        2 YEARS AFTER INTERVENTION

ADJUSTED BY PROPENSITY SCORES PER DISEASE

1120

(YEN/PERSON)

ANNUAL MEDICAL COST

1122
1123
1121
1124
1125

SERVICE A
SERVICE B
NONINTERVENTION

DISEASE 1
DISEASE 2
DISEASE 3

BEFORE INTERVENTION        1 YEAR AFTER INTERVENTION        2 YEARS AFTER INTERVENTION

MEDICAL COST RESTRAINT EFFECTS PER DISEASE

1104

| | SERVICE A | | SERVICE B | |
|---|---|---|---|---|
| | 1 YEAR AFTER | 2 YEARS AFTER | 1 YEAR AFTER | 2 YEARS AFTER |
| DISEASE 1 | $A_{11}$YEN | $A_{12}$YEN | $B_{11}$YEN | $B_{12}$YEN |
| DISEASE 2 | $A_{21}$YEN | $A_{22}$YEN | $B_{21}$YEN | $B_{22}$YEN |
| DISEASE 3 | $A_{31}$YEN | $A_{32}$YEN | $B_{31}$YEN | $B_{32}$YEN |
| TOTAL | $A_1$YEN | $A_2$YEN | $B_1$YEN | $B_2$YEN |

*Fig. 11*

1201 — START

902 — ACQUIRE DATA FOR ANALYSIS

1203 — CALCULATE AVERAGE MEDICAL COST DIFFERENCES

1204 — EXTRACT DISEASES

903 — GENERATE PROPENSITY SCORE CALCULATION EQUATIONS

904 — CALCULATE PROPENSITY SCORES PER DISEASE

905 — CALCULATE ADJUSTED MEDICAL COST PER INTERVENTION SERVICE

906 — CALCULATE ADJUSTED MEDICAL COST FOR NONINTERVENTION GROUP

907 — CALCULATE MEDICAL COST EFFECTS

908 — DISPLAY MEDICAL COST EFFECTS

1209 — END

*Fig. 12*

| 201 INSURED PERSON ID | 1301 HEALTH CHECKUP DATE | 1302 BMI | 1303 FASTING BLOOD SUGAR | 1304 SYSTOLIC BLOOD PRESSURE | 1305 TRIGLYC ERIDE | 1306 SMOKING | 1307 LIFESTYLE IMPROVE-MENT WILL | 142 ... |
|---|---|---|---|---|---|---|---|---|
| K0001 | 2004/05/09 | 25 | 105 | 130 | 150 | PRESENT | PRESENT | |
| K0001 | 2005/05/15 | 24 | 100 | 125 | 130 | ABSENT | ABSENT | |
| K0002 | 2004/05/13 | 22 | 110 | 119 | 90 | PRESENT | PRESENT | |
| K0002 | 2005/05/20 | 21 | 108 | 119 | 90 | ABSENT | ABSENT | |
| K0003 | 2004/06/05 | 25 | 110 | 120 | 150 | ABSENT | ABSENT | |
| K0003 | 2005/06/10 | 23 | 111 | 122 | 155 | PRESENT | PRESENT | |
| K0004 | 2004/02/10 | 20 | 90 | 125 | 140 | PRESENT | PRESENT | |
| K0004 | 2005/02/20 | 20 | 90 | 120 | 130 | ABSENT | ABSENT | |
| ... | | | | | | | | |

HEALTH CHECKUP INFORMATION

Fig. 13

1401 — START

902 — ACQUIRE DATA FOR ANALYSIS

1403 — ACQUIRE HEALTH CHECKUP INFORMATION

1404 — CALCULATE HEALTHCARE ITEM CONTRIBUTIONS

1405 — DETERMINE CONTRIBUTED DISEASES

904 — CALCULATE PROPENSITY SCORES PER DISEASE

905 — CALCULATE ADJUSTED MEDICAL COST PER INTERVENTION SERVICE

906 — CALCULATE ADJUSTED MEDICAL COST FOR NONINTERVENTION GROUP

907 — CALCULATE MEDICAL COST EFFECTS

908 — DISPLAY MEDICAL COST EFFECTS

1409 — END

*Fig. 14*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013104665 A

- JP 2004341611 A